# EUROPEAN PATENT APPLICATION

(11) **EP 2 080 767 A1**
(43) Date of publication of application: **22.07.2009**
(21) Application number: 07831200.6
(22) Date of filing: 05.11.2007
(51) Int. Cl.: C07K 7/00, A61K 9/10, A61K 9/127, A61K 47/34, A61K 47/42, A61K 48/00, C12N 15/09

(54) **COMPOSITION FOR INTRODUCTION OF NUCLEIC ACID**

(30) Priority: 09.11.2006 JP 2006304591
(71) Applicant: Daiichi Sankyo Company, Limited, Chuo-ku Tokyo 103-0023 (JP)
(72) Inventor: KIKUCHI, Hiroshi, Tokyo 134-8630 (JP); HASHIMOTO, Kouichi, Tokyo 134-8630 (JP); KOBAYASHI, Hideo, Tokyo 134-8630 (JP); IIJIMA, Ayako, Tokyo 134-8630 (JP); ASANO, Daigo, Tokyo 134-8630 (JP)
(74) Representative: Arends, William Gerrit
(86) International application number: PCT/JP2007/071467
(87) International publication number: WO 2008/056623

(57) **Abstract**

The present invention provides a composition with weak cytotoxicity for introducing a nucleic acid, such as a short oligonucleotide or a gene, into a cell and expression of the gene in the cell with improved capability for introduction of the nucleic acid, and a novel compound contained in the composition. When a compound represented by a formula (I):

(R¹)n -R²-R³ (I)

wherein (R¹)n represents a polyamino acid residue consisting of a total of n amino acid residues, which are identical to or different from one another, the n residues being selected from the group consisting of an arginine residue, a lysine residue, and a histidine residue, and n being an integer of from 4 to 16; R² represents a single bond or an amino acid residue; and R³ represents a phospholipid residue with 1 or 2 identical or different unsaturated fatty acid residues having from 12 to 20 carbon atoms, and a lipid are administered or supplied to a cell together with a nucleic acid such as a gene, the nucleic acid can be efficiently introduced with weak cytotoxicity.

## Description

### Technical Field

The present invention relates to a compound and a composition for introducing a nucleic acid into a cell.

### Background Art

As methods for introducing a nucleic acid such as a gene into a cell, methods using a cationic lipid solely or a complex formed by a liposome containing the same and a nucleic acid (for example, refer to Patent Document 1) are known. For example, "Lipofectamine," "Lipofectin," "Transfectam," "Genetransfer," "Lipofectamine 2000," and the like are marketed as reagents used for this method.

However, the following problems have been pointed out about these commercially available reagents. a) Storage stability as a formulation is poor, and the reproducibility of the introduction of a gene into a cell using a liposome or the like and the expression of the gene poor. b) Since these reagents are very unstable in serum to be added to a medium for cell culture (fetal bovine serum), a complicated procedure is used comprising replacing a serum-containing medium in which cells are cultured, with a serum-free medium, and then, after introduction, returning the cells to a serum-containing medium. Recently, it has been revealed that these reagents are very unstable in blood or in the body. c) Many of commercially available products (for example, Lipofectamine, Lipofectin, and Lipofectamine 2000) are provided only in the form in which a lipid has already been dispersed in water and require a procedure of adding an aqueous gene solution externally to this form. In this form, however, a complex with a gene binding to the outside of a liposome can be produced, but a liposome with a gene included therein cannot be produced. Furthermore, Lipofectamine 2000 requires cumbersome procedures requiring special caution since it cannot be excessively stirred or shaken to avoid peroxide formation of a cationic lipid. d) Cytotoxicity is very strong.

Thus, several reagents for introducing a nucleic acid such as a gene into a cell using a cationic lipid solely or a liposome are commercially available but suffer from many problems.
Patent Document 1: Japanese Patent Laid-Open No. 2-135092

### Disclosure of the Invention

### Problems to be Solved by the Invention

An object of the present invention is to provide a composition with weak cytotoxicity for introducing a nucleic acid, such as a short oligonucleotide or a gene, into a cell and expression of the gene in the cell with improved introduction of the nucleic acid, and a novel compound contained in the composition.

### Means for Solving the Problems

The inventors of the present invention conducted various researches about methods for improving cytotoxicity, the capability for the introduction of a nucleic acid into a cell, and the expression of the gene in the cell. As a result, they found that a nucleic acid can be efficiently introduced with weak cytotoxicity by administering or supplying to a cell a compound represented by the following formula (I):

(R¹)n -R²-R³ (I)

wherein: (R¹)n represents a polyamino acid residue consisting of a total of n amino acid residues, which are identical to or different from one another, the n residues being selected from the group consisting of an arginine residue, a lysine residue, and a histidine residue, and n being an integer of from 4 to 16; R² represents a single bond or an amino acid residue, and R³ represents a phospholipid residue with 1 or 2 identical or different unsaturated fatty acid residues having from 12 to 20 carbon atoms, or a salt thereof, and a lipid together with a nucleic acid such as a gene, and thereby accomplished the present invention.

Specifically, the present invention relates to the following inventions.
(1) A compound represented by a formula (I):

   (R¹)n -R²-R³ (I)

   wherein: (R¹)n represents a polyamino acid residue consisting of a total of n amino acid residues, which are identical to or different from one another, the n residues being selected from the group consisting of an arginine residue, a lysine residue, and a histidine residue; and n being an integer of from 4 to 16; R² represents a single bond or an amino acid residue, and R³ represents a phospholipid residue with 1 to 4 identical or different unsaturated fatty acid residues having from 12 to 20 carbon atoms, or a salt thereof.
(2) The compound or a salt thereof according to the above (1), wherein R¹ are all arginine residues.
(3) The compound or a salt thereof according to the above (1) or (2), wherein n is from 7 to 11.
(4) The compound or a salt thereof according to the above (1) or (2), wherein n is from 8 to 10.
(5) The compound or a salt thereof according to any one of the above (1) to (4), wherein R² is a glycine residue.
(6) The compound or a salt thereof according to any one of the above (1) to (5), wherein R³ is a dioleoyl phosphatidyl ethanolamine residue.
(7) A composition comprising the compound or a salt thereof according to any one of the above (1) to (6) and a lipid.
(8) The composition according to the above (7), wherein the lipid is a phospholipid and/or a sterol.
(9) The composition according to the above (8), wherein the phospholipid is one or more selected from phosphatidyl ethanolamines, phosphatidylcholines, phosphatidylserines, phosphatidylinositols, phosphatidylglycerols, cardiolipins, sphingomyelins, plasmalogens, and phosphatidic acids.
(10) The composition according to the above (8), wherein the phospholipid is a phosphatidyl ethanolamine.
(11) The composition according to the above (8), wherein the phospholipid is a dioleoyl phosphatidyl ethanolamine.
(12) The composition according to any one of the above (8) to (11), wherein the sterol is cholesterol.
(13) The composition according to any one of the above (7) to (12), further comprising a cationic substance.
(14) The composition according to the above (13), wherein the cationic substance is one or more selected from poly L-lysine, protamine or a salt thereof, pronectin, and spermine.
(15) The composition according to any one of the above (7) to (14), for introducing a nucleic acid into a cell.
(16) The composition according to any one of the above (7) to (15), further comprising a nucleic acid.
(17) The composition according to any one of the above (7) to (16), which forms a lipid membrane structure.
(18) The composition according to the above (17), wherein the lipid membrane structure is a liposome.
(19) A method for introducing a nucleic acid into a cell, **characterized in that** the composition according to any one of the above (16) to (18) is applied to a cell *in vitro* or *in vivo*.

### Advantage(s) of the Invention

As shown in the examples described later, a nucleic acid can be efficiently introduced into a cell with weak cytotoxicity using the compound represented by the formula (I) of the present invention. Therefore, the composition of the present invention is useful as a reagent or a medicament for introducing a nucleic acid.

### Best Mode for Carrying Out the Invention

The composition of the present invention is a composition that is used together with a nucleic acid to be introduced in order to introduce the nucleic acid into a cell. It is sufficient that it contains at least the compound represented by the above-mentioned formula (I) and a lipid, and those compositions containing a nucleic acid to be introduced (nucleic acid-containing compositions) and those compositions not containing a nucleic acid fall within the scope of the present invention.

Substituents in the compound represented by the formula (I) of the present invention will be explained below.

In the formula (I), (R¹)n represents a polyamino acid residue consisting of a total of n amino acid residues, which are identical to or different from one another, the n residues are selected from the group consisting of an arginine residue, a lysine residue, and a histidine residue, and n is an integer of from 4 to 16, and amino acid residues in the polyamino acid residue may be bound by block polymerization or random polymerization. When R¹ are all arginine residues, this represents a polyarginine residue. n represents an integer of from 4 to 16, preferably 7, 8, 9, 10, or 11, particularly preferably 8, 9, or 10.

R² represents a single bond or an amino acid residue. The amino acid residue is not particularly limited, and examples thereof include a glycine residue, an alanine residue, and so forth. Of these, a glycine residue is more preferred.

R³ represents a phospholipid residue with 1 to 4 identical or different unsaturated fatty acid residues having from 12 to 20 carbon atoms. The phospholipid includes phosphatidyl ethanolamines, phosphatidylcholines, phosphatidylserines, phosphatidylinositols, phosphatidylglycerols, cardiolipins, sphingomyelins, ceramide phosphoryl ethanolamines, ceramide phosphorylglycerols, ceramide phosphorylglycerol phosphates, 1,2-dimyristoyl-1,2-deoxyphosphatidylcholines, plasmalogens, phosphatidic acids, and so forth. The unsaturated fatty acid having from 12 to 20 carbon atoms includes oleic acid, elaidic acid, linoleic acid, linolenic acid, stearolic acid, arachidonic acid, and so forth. The phospholipid with 1 to 4 identical or different unsaturated fatty acid residues having from 12 to 20 carbon atoms includes phospholipids having 1 or 2 acyl groups derived from these unsaturated fatty acids. Of these, as the phospholipids, phosphatidyl ethanolamines are preferred, and those having 2 unsaturated fatty acid residues are more preferred. Of these, dioleoyl phosphatidyl ethanolamine (DOPE) is particularly preferred.

Methods for producing the compound represented by the formula (I) of the present invention are not particularly limited. When R² represents a single bond, the compound can be produced by binding the above-mentioned polyamino acid and the above-mentioned phospholipid. When R² represents one amino acid residue that serves as a linker, the compound can be produced by synthesizing a peptide obtained by binding the above-mentioned polyarginine and a linker amino acid and then binding the above-mentioned phospholipid to the linker amino acid.

In general, a phospholipid and a polyamino acid or a linker are bound by reacting an amino group or a carboxyl group at an end of the polyamino acid or the linker and a reactive functional group of the phospholipid (for example, a hydroxyl group, a carboxyl group, an ester group, an amino group, etc.). A hydroxyl group already existing in the phospholipid may be used as a reactive functional group. For example, a carboxyl group can also be used as a reactive functional group by a method of introducing a carboxyl group into a phospholipid using a technique of oxidizing a hydroxyl group in the phospholipid to a carboxyl group, introducing a functional group including a carboxyl group into a phospholipid, or the like and further esterifying the carboxyl group, if necessary.

The compound represented by the formula (I) of the present invention or a compound in which a phospholipid and a linker are bound can be produced by reacting a reactive functional group such as a carboxyl group or an ester group of a phospholipid and an amino group of a polyamino acid or a linker to form an amide bond, or reacting a reactive functional group such as an amino group of a phospholipid and a carboxy group of a polyamino acid or a linker to form an amide bond. For this reaction, common methods such as an acid halide method, an active ester method, and an acid anhydrate method can be used.

In the acid halide method, a target compound can be obtained by treating a phospholipid having a carboxyl group with a halogenating agent in an inert solvent to obtain an acid halide and then reacting the obtained acid halide and an amino group of a polyamino acid or a linker or by treating a carboxyl group of a polyamino acid or a linker with a halogenating agent to obtain an acid halide and then reacting the obtained acid halide and an amino group of a phospholipid.

The types of solvents are not particularly limited so long as they are used in reactions for producing acid halides, and it is sufficient that they dissolve a starting material without inhibiting the reaction. Preferred examples thereof include ethers such as diethyl ether, tetrahydrofuran, and dioxane, amides such as dimethylformamide, dimethylacetamide, and hexamethyl phosphoric triamide, halogenated hydrocarbons such as dichloromethane, chloroform, and 1,2-dichloroethane, nitriles such as acetonitrile and propionitrile, esters such as ethyl formate, ethyl acetate, and mixed solvents thereof. Examples of halogenating agents include thionyl halides such as thionyl chloride, thionyl bromide, and thionyl iodide, sulfuryl halides such as sulfuryl chloride, sulfuryl bromide, and sulfuryl iodide, trihalogenated phosphoruses such as phosphorus trichloride, phosphorus tribromide, and phosphorus triiodide, pentahalogenated phosphoruses such as phosphorus pentachloride, phosphorus pentabromide, and phosphorus pentiodide, oxyhalogenated phosphoruses such as phosphorus oxychloride, phosphorus oxybromide, and phosphorus oxyiodide, halogenated oxalyls such as oxalyl chloride and oxalyl bromide, and so forth. The reaction can be performed at between 0°C and the reflux temperature of the solvent, between room temperature and the reflux temperature of the solvent being preferred.

Solvents used in a reaction of the obtained acid halide and an amino group of a polyamino acid or a phospholipid are not particularly limited so long as they dissolve a starting material without inhibiting the reaction. Examples thereof include ethers such as diethyl ether, tetrahydrofuran, and dioxane, amides such as dimethylformamide, dimethylacetamide, hexamethylphosphoric triamide, esters such as ethyl formate and ethyl acetate, sulfoxides such as dimethyl sulfoxide, and mixed solvents thereof. In a reaction of an acid halide and an amino group of a polyamino acid or a phospholipid, an organic base such as triethylamine or pyridine can also be added, if necessary.

Active esterification is performed by allowing a phospholipid or a polyamino acid having a carboxyl group or a linker to react with an active esterification agent in a solvent to produce an active ester and then allowing the active ester to react with an amino group of the polyamino acid or the linker or an amino group of the phospholipid. Examples of the solvent include halogenated hydrocarbons such as methylene chloride, chloroform, ethers such as ether and tetrahydrofuran, amides such as dimethylformamide and dimethylacetamide, aromatic hydrocarbons such as benzene, toluene, and xylene, esters such as ethyl acetate, and mixed solvents thereof. Examples of the active esterification agent include N-hydroxy compounds such as N-hydroxysuccimide, 1-hydroxybenzotriazole, and N-hydroxy-5-norbornene-2,3-dicarboxyimide; diimidazole compounds such as 1,1'-oxalyldiimidazole and N,N'-carbonyldiimidazole; disulfide compounds such as 2,2'-dipyridyldisulfide; succinic acid compounds such as N,N'-disuccinimidyl carbonate; phosphinic chloride compounds such as N,N'-bis(2-oxo-3-oxazolidinyl)phosphinic chloride; oxalate compounds such as N,N'-disuccinimidyl oxalate (DSO), N,N'-diphthalimide oxalate (DPO), N,N'-bis(norbornenylsuccinimidyl)oxalate (BNO), 1,1'-bis(benzotriazolyl)oxalate (BBTO), 1,1'-bis(6-chlorobenzotriazolyl)oxalate (BCTO), 1,1'-bis(6-trifluoromethylbenzotriazolyl)oxalate (BTBO), and so forth.

A phospholipid or an amino group of a polyamino acid or a linker is preferably allowed to react with an active ester in the presence of a condensing agent such as, for example, lower dialkyl azodicarboxylate-triphenylphosphines such as diethyl azodicarboxylate-triphenylphosphine, N-lower alkyl-5-arylisooxazolium-3'-sulfonates such as N-ethyl-5-phenylisooxazolium-3'-sulfonate, oxydiformates such as diethyloxydiformate (DEPC), N',N'-dicycloalkylcarbodiimides such as N',N'-dicyclohexylcarbodiimide (DCC), diheteroaryldiselenides such as di-2-pyridyldiselenide, triarylphosphines such as triphenylphosphine, arylsulfonyltriazolides such as p-nitrobenzenesulfonyltriazolide, 2-halo-1-lower alkyl pyridinium halide such as 2-chloro-1-methylpyridinium iodide, diarylphosphoryl azides such as diphenylphosphoryl azide (DPPA), imidazole derivatives such as N,N'-carbodiimidazole (CDI), benzotriazole derivatives such as 1-hydroxybenzotriazole (HOBT), dicarboxyimide derivatives such as N-hydroxy-5-norbornene-2,3-dicarboxyimide (HONB), carbodiimide derivatives such as 1-ethyl-3-(3-dimethylaminopropyl)carbodiimide (WSC), phosphonic acid cyclic anhydrates such as 1-propanephosphonic acid cyclic anhydrate (T3P). The temperature for the reaction for preparing an active ester is from -10°C to room temperature, and the reaction of an active ester compound with a phospholipid or with an amino group of a polyamino acid or a linker is performed at around room temperature. The reaction time is approx. from 30 min to 10 h for both the reactions.

The mixed acid anhydride method is implemented by producing a mixed acid anhydride of a phospholipid or a carboxyl group of a polyamino acid or a linker and then reacting the polyamino acid or the linker or an amino group of the phospholipid. The reaction for producing a mixed acid anhydride can be performed in an inactive solvent (for example, ethers such as ethers and tetrahydrofuran and amides such as dimethylformamide and dimethylacetamide) using lower alkyl halide carbonates such as ethyl chlorocarbonate and isobutyl chlorocarbonate, lower dialkyl cyanophosphoric acids such as diethylcyanophosphoric acid, and the like. The reaction is preferably performed in the presence of an organic amine such as triethylamine or N-methylmorpholine. The reaction temperature is from -10°C to room temperature. The reaction time is approx. from 30 min to 5 h. A reaction of the mixed acid anhydride with a polyamino acid or a linker or with an amino group of a phospholipid is preferably performed in an inactive solvent (for example, ethers such as ethers and tetrahydrofuran and amides such as dimethylformamide and dimethylacetamide) in the presence of the above-mentioned organic amines. The reaction temperature is from 0°C to room temperature, and the reaction time is approx. from 1 to 24 h. Furthermore, condensation can also be performed by directly reacting a compound having a carboxyl group and a compound having an amino group in the presence of the above-mentioned condensing agents. This reaction can be performed in the same manner as in the above-mentioned reaction for producing an active ester.

Subsequently, a phospholipid derivative of the composition of the present invention can be obtained by reacting a reactive functional group of the obtained linker-bound polyamino acid (for example, a carboxyl group, a hydroxyl group, etc.) and a reactive functional group of a phospholipid compound (for example, a carboxyl group, an amino group, etc.). For example, when phosphatidyl ethanolamine is used as a phospholipid compound, a phospholipid derivative of the present invention can be produced by reacting an amino group of the phospholipid compound and a carboxyl group at the linker end of the linker-bound polyamino acid. This reaction can be performed in the same manner as in the reaction described above, and is preferably performed by an active ester method or the like in the presence of a condensing agent.

In each of the above-mentioned reactions, a target reaction may be efficiently performed by introducing a protective group. A protective group can be introduced with reference to, for example, "Protective Groups in Organic Synthesis" (P.G.M. Wuts and T. Green, 3rd ed., 1999, Wiley, John & Sons) or the like. A target compound can be isolated and purified by a usual method employed in this field. For example, purification by high performance liquid chromatography or the like is preferred. Substances in the form of salts of the phospholipid derivatives of the present invention also fall within the scope of the present invention. Types of salts are not particularly limited, and examples thereof include mineral acid salts such as hydrochlorides and sulfates, organic acid salts such as oxalates and acetates, metal salts such as sodium salts and potassium salts, organic amine salts such as ammonium salts and methyl amine salts, and so forth.

In the present invention, a compound is particularly preferred in which all R¹ in (R¹)n in the general formula (I) represent arginine residues, (R¹)n represents a polyarginine residue with n being 8, R² represents a single bond or a glycine residue, and R³ represents a dioleoyl phosphatidyl ethanolamine residue.

In the composition of the present invention, the content of the compound represented by the general formula (I) can be suitably determined depending on the total lipid content in the composition of the present invention, the type of nucleic acid to be introduced, the purpose for use, the form of the composition, and the like, and is preferably 0.5 to 50% by mole, more preferably 1 to 10% by mole to the total lipid content.

Examples of lipids used in the composition of the present invention include phospholipids such as phosphatidyl ethanolamines, phosphatidylcholines, phosphatidylserines, phosphatidylinositols, phosphatidylglycerols, cardiolipins, sphingomyelins, plasmalogens, and phosphatidic acids, sterols such as cholesterol and cholestanol, and so forth. One of these lipids or two or more in combination can be used. Of these, phospholipids and sterols are preferably used in combination. Phosphatidyl ethanolamines as phospholipids and sterols are more preferably used in combination. Among these lipids, a fatty acid residue in a phospholipid is not particularly limited, and examples thereof include saturated or unsaturated fatty acid residues having from 12 to 18 carbon atoms. A myristoyl group, a palmitoyl group, a stearoyl group, an oleoyl group, a linoleyl group, and the like are preferred.

The amount of the above-mentioned lipid mixed in the composition of the present invention can be suitably determined depending on the total lipid content. The amount of phospholipids included in the compound represented by the general formula (I) is preferably from 50 to 100% by mole, more preferably from 60 to 100% by mole of the total lipid content. Further, the amount of sterols is preferably from 0 to 50% by mole, more preferably from 0 to 40% by mole of the total lipid content.

As a nucleic acid to be introduced into the composition of the present invention, any of the following can be used: oligonucleotides, DNAs, RNAs, nucleic acids including both deoxyribose and ribose, phosphoric acid derivatives such as phosphothioates and boranophosphates, and/or synthetic artificial nucleic acids including sugars subjected to chemical modifications such as 4'-thioation, 2'-O-methylation, 2'-O-methoxyethylation, 2'-amination, 2'-fluorination, 2-hydroxyethylphosphation, crosslinking at the 2' and 5' positions by an ether bond and/or chemically modified bases such as 2,6-diaminopurine, 5-bromouridine, 5-iodouridine, 4-thiouridine, N-3-methyluridine, and 5-(3-aminoallyl)uridine, and functional artificial nucleic acids to which PEG, antibodies, membrane-permeable peptides or lipids bind directly. Examples thereof include short oligonucleotides such as antisense oligonucleotides, antisense DNAs, decoy nucleic acids, antisense RNAs, shRNAs, siRNAs, miRNAs, bioactive substances such as enzymes and cytokines, genes coding for antisense RNAs, shRNAs, siRNAs, and miRNAs, peptide nucleic acids, and so forth.

In the present invention, to introduce a nucleic acid into a cell efficiently, the negative electric charge of a nucleic acid may be regulated. Examples of regulation methods include methods comprising forming a complex with the nucleic acid using a cationic substance such as poly L-lysine (PLL), protamine or a salt thereof, pronectin, or spermine. For example, the substance preferably contains from 2.0 to 4.8 moles, more preferably from 2.2 to 3.6 moles of PLL in nitrogen count equivalent based on 1 mole of nucleic acid being equivalent to a phosphate group. Alternatively, the substance preferably contains from 0.6 to 2.6 mg, more preferably from 0.9 to 1.9 mg of protamine based on 1 mg of a nucleic acid. When a complex of a cationic substance and a nucleic acid has a positive electric charge partially and/or overall, the complex can be efficiently carried by a lipid composition containing a negative electric charge lipid. Examples of such negatively charged lipids include lipids such as cholesteryl hemisuccinate (CHEMS) and dimyristoyl phosphatidyl glycerol (DMPG). The amount thereof mixed in the lipid composition can be suitably determined depending on the total lipid content and is preferably from 0 to 50% by mole, more preferably from 5 to 40% by mole.

The composition of the present invention may be a lipid membrane structure containing the compound represented by the general formula (I) and a phospholipid alone, a lipid membrane structure containing the compound represented by the general formula (I), a phospholipid, and a sterol in combination, and a lipid membrane structure further containing other components in addition to a sterol.

The form of the lipid membrane structure is not particularly limited, and examples thereof include a dried lipid mixture form, a form in which a lipid membrane structure is dispersed in an aqueous medium, a form in which this form is dried or frozen, and so forth.

Of these, examples of the form in which a lipid membrane structure is dispersed in an aqueous medium include a one-layer membrane liposome, a multilayer liposome, an O/W emulsion, a W/O/W emulsion, a spherical micelle, a wormlike micelle, an amorphous layered structure, and so forth. Of these, a liposome is preferred. The size of a lipid membrane structure in the dispersion is not particularly limited. For example, the particle size of a liposome or an emulsion is from 50 nm to 5 µm. The particle size of a spherical micelle is from 5 to 100 nm. When a wormlike micelle or an amorphous layered structure is used, the thickness per layer is from 5 to 10 nm, and these layers preferably form a multilayer structure.

Various lipid membrane structures and preparation examples thereof will be explained below.
1) A lipid membrane structure in the form of a dried mixture can be produced by, for example, dissolving all the components of the lipid membrane structure or a part thereof in an organic solvent such as chloroform first and then drying the mixture with an evaporator under reduced pressure or spray drying the mixture with a spray dryer.
2) The form in which a lipid membrane structure is dispersed in an aqueous medium can be produced by adding the above-mentioned dried mixture to an aqueous medium and further emulsifying the mixture using an emulsifier such as a homogenizer, an ultrasonic emulsifier, and a high-pressure jet emulsifier. Furthermore, this form can also be produced by methods known as methods for producing a liposome such as, for example, a reverse-phase evaporation method. When the size of the lipid membrane structure is to be regulated, extrusion (extrusion filtration) can be performed under high pressure using a membrane filter of a uniform pore size. In addition, a lipid membrane structure having a novel membrane composition and/or a membrane structure can be produced by further adding the compound represented by the general formula (I), a lipid such as a phospholipid or a sterol, and the like to the lipid membrane structure during or after emulsification.
   The composition of the aqueous medium (dispersion medium) is not particularly limited, and examples thereof include buffers such as a phosphate buffer, a citrate buffer, and a phosphate-buffered physiological saline, physiological saline, a cell culture medium, and so forth. A lipid membrane structure can be stably dispersed in these aqueous media (dispersion media). Furthermore, sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose, and melezinose, polysaccharides such as cyclodextrin, sugar alcohols such as erythritol, xylitol, sorbitol, mannitol, and maltitol, polyhydric alcohols (aqueous solutions) such as glycerine, diglycerine, polyglycerine, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol, and the like may be added. To store this lipid membrane structure dispersed in the aqueous medium (dispersion medium) stably for a long period, it is recommended to minimize electrolytes in the aqueous medium (dispersion medium) in view of physical stability such as prevention of agglutination. Furthermore, in view of chemical stability of the lipid, it is recommended to set the pH of the aqueous medium (dispersion medium) in the range from weak acidic to around neutral conditions (pH 3.0 to 8.0) and to remove dissolved oxygen by nitrogen bubbling.
   Here, the concentration of a sugar or a polyhydric alcohol is not particularly limited. Where a lipid membrane structure is dispersed in an aqueous medium, for example, the concentration of a sugar (aqueous solution) is preferably from 2 to 20% (W/V), more preferably from 5 to 10% (W/V). Furthermore, the concentration of a polyhydric alcohol (aqueous solution) is preferably from 1 to 5% (W/V), more preferably from 2 to 2.5% (W/V). When a buffer is used as an aqueous medium (dispersion medium), the concentration of the buffer is preferably from 5 to 50 mM, more preferably from 10 to 20 mM. The concentration of a lipid membrane structure in an aqueous medium (dispersion medium) is not particularly limited, but the concentration of all lipids contained in a lipid membrane structure is preferably from 500 mM or less, more preferably from 0.001 to 100 mM.
3) The form in which a lipid membrane structure that is dispersed in an aqueous medium is dried or frozen can be produced by drying or freezing the above-mentioned lipid membrane structure dispersed in the aqueous medium with usual lyophilization and spray drying. When a lipid membrane structure in the form dispersed in an aqueous medium is produced first and then dried, the lipid membrane structure can be stored for a long period. In addition, there is an advantage that, when a nucleic acid-containing aqueous solution is added to this dried lipid membrane structure, the lipid mixture is efficiently hydrated. Therefore, a nucleic acid can be efficiently carried inside the lipid membrane structure.

When lyophilization or spray drying is performed, the structures can be stored stably over a long period, for example, using sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose and melezinose, polysaccharides such as cyclodextrin, and sugar alcohols such as erythritol, xylitol, sorbitol, mannitol, and maltitol. Furthermore, when they are frozen, the structures can be stored stably over a long period, for example, using each of the above-mentioned sugars (aqueous solutions) and polyhydric alcohols (aqueous solutions) such as glycerine, diglycerine, polyglycerine, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol. Sugars and polyhydric alcohols may be used in combination.

The composition of the present invention can be a composition containing a nucleic acid to be introduced (nucleic acid-containing composition). The composition in this case will be explained.

The form of the composition may exist simply as a mixture containing the compound represented by the general formula (I), a phospholipid, and a nucleic acid, as well as a sterol as desired in addition to the above-mentioned components, or in the form of a mixture of a lipid membrane structure formed by the compound represented by the general formula (I), a phospholipid, a sterol, and the like in combination and a nucleic acid. Alternatively, a form in which a nucleic acid is carried by a lipid membrane structure may be used. Here, the expression "carried" means that a nucleic acid exists in the lipid membrane of the lipid membrane structure, on the surface or inside thereof, in the lipid layer and/or on the surface of the lipid layer. When a lipid membrane structure is, for example, a microparticle such as a liposome, a nucleic acid can be included in the microparticle.

Furthermore, examples of the form of the lipid membrane structure include a mixed dry matter form, a form dispersed in an aqueous medium, and a form in which this form is further dried or frozen, and so forth, as in the case of the above-mentioned lipid membrane structure.

Various nucleic acid-containing lipid membrane structures and preparation examples thereof will be explained below.
1) When a mixed dry matter form is used, for example, a lipid membrane structure can be produced by dissolving components of the lipid membrane structure and a nucleic acid in an organic solvent such as chloroform to obtain a mixture first and then subjecting this mixture to drying under reduced pressure using an evaporator or spray drying this mixture with a spray dryer.
2) Several production methods for producing a form dispersed in an aqueous medium containing a lipid membrane structure and a nucleic acid are known, and a suitable production method such as the following methods 2-1 to 2-5 can be selected depending on the mode of carrying the nucleic acid in the lipid membrane structure, properties of the mixture, and the like.
   2-1) Production Method 1
      In this production method, an aqueous medium is added to the above-mentioned mixed dry matter, and the mixture is further emulsified using an emulsifier such as a homogenizer, an ultrasonic emulsifier, a high-pressure jet emulsifier, or the like. When the size (particle size) is to be regulated, a membrane filter of a uniform pore size can be further used for extrusion (extrusion filtration) under high pressure. In this method, to obtain a mixed dry matter containing components of a lipid membrane structure and a nucleic acid, the lipid membrane structure and the nucleic acid need to be dissolved in an organic solvent first. This method has an advantage that best use can be made of interactions between components of the lipid membrane structure and the nucleic acid. Specifically, when a lipid membrane structure has a layered structure, a nucleic acid can penetrate into the multilayer, and this production method has an advantage of increasing the rate of carrying the nucleic acid by the lipid membrane structure.
   2-2) Production Method 2
      In this production method, a dry matter obtained by dissolving components of a lipid membrane structure in an organic solvent first and then evaporating the organic solvent is emulsified by further adding an aqueous medium containing a nucleic acid. When the size (particle size) is to be regulated, extrusion (extrusion filtration) can be further performed under high pressure using a membrane filter of a uniform pore size. This method can be applied to a nucleic acid that is hardly soluble in an organic solvent but can be dissolved in an aqueous medium. When the lipid membrane structure is a liposome, there is an advantage that a nucleic acid can be carried in an aqueous phase portion therein.
   2-3) Production Method 3
      In this production method, an aqueous medium containing a nucleic acid is further added to a lipid membrane structure already dispersed in an aqueous medium, such as a liposome, an emulsion, a micelle, or a layered structure. A water-soluble nucleic acid can be used as a target. A nucleic acid is added to the already produced lipid membrane structure externally in this method. Therefore, in the case of a high-molecular-weight nucleic acid, the nucleic acid cannot penetrate into the lipid membrane structure and may exist on (bind to) the surface of the lipid membrane structure. When a liposome is used as a lipid membrane structure, it is known that a sandwich structure (generally referred to as a complex) is formed with a nucleic acid sandwiched between liposome particles by this Production Method 3. In this production method, since a water dispersion containing a lipid membrane structure alone is produced beforehand, decomposition of the nucleic acid in emulsification or the like does not need to be taken into account, and the size (particle size) can be easily regulated. Therefore, production is relatively easy as compared with Production Method 1 or 2.
   2-4) Production Method 4
      In this production method, an aqueous medium containing a nucleic acid is further added to dry matter obtained by producing a lipid membrane structure dispersed in an aqueous medium and drying the structure. As in Production Method 3, a water-soluble nucleic acid can be used as a target nucleic acid. A difference from Production Method 3 is in the mode of existence of the lipid membrane structure and the nucleic acid. Since a lipid membrane structure dispersed in an aqueous medium is produced first and then further dried to produce a dry matter in this Production Method 4, the lipid membrane structure at this stage exists in a solid state as a lipid membrane fragment. To allow this lipid membrane fragment to exist in a solid state, a solvent further containing a sugar (aqueous solution), preferably sucrose (aqueous solution) or lactose (aqueous solution) is preferably used as an aqueous medium as described above. Here, when an aqueous medium containing a nucleic acid is added, a lipid membrane fragment existing in a solid state starts being hydrated rapidly along with the entry of water, and the lipid membrane structure can be reconstructed. At this time, a structure in the form in which the nucleic acid is carried inside the lipid membrane structure can be produced.
      In Production Method 3, in the case of a high-molecular-weight nucleic acid, the nucleic acid exists in the mode that the nucleic acid cannot penetrate into the lipid membrane structure and binds to the surface of the lipid membrane structure. Production Method 4 differs greatly at this point. Specifically, a part or all of each nucleic acid is taken up into the lipid membrane structure. Since a dispersion of a lipid membrane structure alone is produced beforehand in this Production Method 4, decomposition of a nucleic acid during emulsification does not need to be taken into account, and the size (particle size) can be regulated easily. Therefore, production is relatively easy as compared with Production Methods 1 and 2. In addition, since lyophilization or spray drying is performed first, there are advantages that storage stability as a formulation (nucleic acid-containing composition) is easily ensured, that the size (particle size) can be recovered when a dried formulation is rehydrated with an aqueous solution of a nucleic acid, that even a polymer nucleic acid can be easily carried inside the lipid membrane structure, and so forth.
   2-5) Others
      As other methods for producing the form in which a mixture of a lipid membrane structure and a nucleic acid are dispersed in an aqueous medium, methods known as methods for producing a liposome such as, for example, a reverse-phase evaporation method can be employed. When the size (particle size)is to be regulated, extrusion (extrusion filtration) can be performed under high pressure using a membrane filter of uniform pore size.
3) To produce a form obtained by further drying the above-mentioned dispersion in which a mixture of a lipid membrane structure and a nucleic acid are dispersed in an aqueous medium, methods such as lyophilization or spray drying can be employed. At this time, the above-mentioned solvent containing a sugar (aqueous solution), preferably sucrose (aqueous solution) or lactose (aqueous solution) is preferably used as an aqueous medium. Examples of methods for further freezing a dispersion in which a mixture of a lipid membrane structure and a nucleic acid is dispersed in an aqueous medium include usual freezing methods. In this case, a solvent containing a sugar (aqueous solution) or a polyhydric alcohol (aqueous solution) is preferably used as an aqueous medium.
4) A nucleic acid-containing lipid membrane structure having a novel membrane composition and/or a membrane structure can be produced by further adding the compound represented by the general formula (I) and a lipid such as a phospholipid or a sterol to the lipid membrane structure in any of Production Methods 1) to 3) or at any stage of each production method.
5) In any of the Production Methods 1) to 4), a nucleic acid to be introduced may be introduced alone or in the form of a complex with a cationic substance.

When the composition of the present invention obtained as described above is used, a nucleic acid can be efficiently introduced into a cell. For example, a nucleic acid can be introduced into a target cell *in vitro* by methods comprising adding the nucleic acid-containing composition of the present invention to a suspension containing the target cell, culturing the target cell in a medium containing the nucleic acid-containing composition, and the like. Furthermore, the nucleic acid-containing composition of the present invention can be administered to a human or a non-human animal *in vivo*. The administration method may be oral or parenteral administration. Common dosage forms for oral administration can be used, and examples thereof include a tablet, a powder, a granule, and so forth. Common dosage forms for parenteral administration can be used, and examples thereof include by injection, an eye drop, an ointment, a suppository, and so forth. Parenteral administration is preferred. Of the dosage forms, injection is more preferred. As the administration method, intravenous injection, in particular, local injection targeting a cell or an organ, is preferred.

### Examples

Hereafter, examples will be described, but the scope of the present invention is not limited to these examples. Example 1 Synthesis of the compound represented by formula (I) [(Arg)8-Gly-DOPE]

Using a Boc-Gly-PAM resin (0.5 mmol) as a starting material, a protected peptide resin Boc-[Arg(Tos)]8-Gly-PAM resin was synthesized by the Boc method using an ABI430A-type Fully Automatic Peptide Solid Phase Synthesizer.

The resulting protected peptide resin was treated with anhydrous hydrogen fluoride, removed from the resin, and deprotected to obtain a crude peptide H-(Arg)8-Gly-OH. The resulting crude peptide was purified by reverse-phase HPLC and Boc-protected to form Boc-(Arg)8-Gly-OH.

The above-mentioned Boc-(Arg)8-Gly-OH and DOPE were condensed with water-soluble carbodiimide in the presence of HOBt, and then the Boc group was removed with TFA to obtain crude H-(Arg)8-Gly-DOPE.

The resulting crude peptide was eluted by the gradient for purification in the 0.1% TFA-containing H₂0-CH₃CN system using a reverse-phase HPLC column (ODS). Fractions containing the target compound with high purity were collected and lyophilized to obtain the target peptide H-(Arg)8-Gly-DOPE.

### Preparation Example 1

Dioleoyl phosphatidyl ethanolamine (DOPE; NOF Corporation) and cholesteryl hemisuccinate (CHEMS; Sigma-Aldrich) were dissolved in chloroform at concentrations of 2.1 and 0.45 mM, respectively, and the mixture was dried under reduced pressure using a rotary evaporator to obtain a lipid mixture. Meanwhile, an aqueous solution of siRNA of the following sequences (1.0 µM) and an 18% sucrose solution containing 290 nM poly-L-lysine hydrobromide (PLL; Sigma-Aldrich) were mixed in a volume ratio of 1:1, and the mixture was incubated at room temperature for 20 min to obtain an siRNA/PLL complex solution.
sense 5'- ACAUCACGUACGCGGAAUACUUCGA -AG - 3' (SEQ ID NO: 1)
antisense 3'-UA- UGUAGUGCAUGCGCCUUAUGAAGCU -5' (SEQ ID NO: 2)

This complex solution and F-12HAM Medium (Sigma) were added to the above-mentioned lipid mixture, and the mixture was incubated at room temperature for 20 min and subjected to ultrasonic irradiation using a sonicator for 1 min with heating at approx. 65°C to obtain polyarginine-non-modified liposome dispersions having the concentrations shown in Table 1 (LP1, 2, 3, and 4). In addition, aqueous solutions of 7.0, 42, 84, and 168 µM R8-G-DOPE were added to LP1, 2, 3, and 4, respectively, in a volume ratio of 13:1, and the mixtures were incubated at 37°C for 30 min to obtain polyarginine-modified liposome dispersions having the concentrations shown in Table 2 (Prescription Examples 1, 2, 3, and 4).

Meanwhile, controls for evaluation were prepared using water instead of the above-mentioned aqueous siRNA solutions.

**[Table 1]**

| Polyarginine-non-modified liposome dispersion | LP1 | LP2 | LP3 | LP4 |
|---|---|---|---|---|
| DOPE concentration (µM) | 8.9 | 53 | 107 | 213 |
| CHEMS concentration (µM) | 2.0 | 12 | 23 | 47 |
| siRNA concentration (nM) | 80 | 80 | 80 | 80 |
| PLL concentration (nM) | 23 | 23 | 23 | 23 |

**[Table 2]**

| Polyarginine-modified liposome dispersion | Prescription Example 1 | Prescription Example 2 | Prescription Example 3 | Prescription Example 4 |
|---|---|---|---|---|
| DOPE concentration (µM) | 8.2 | 49 | 99 | 198 |
| CHEMS concentration (µM) | 1.8 | 11 | 22 | 43 |
| R₈-G-DOPE concentration (µM) | 0.50 | 3.0 | 6.0 | 12 |
| siRNA concentration (nM) | 74 | 74 | 74 | 74 |

### Preparation Example 2

Dioleoyl phosphatidyl ethanolamine (DOPE; NOF Corporation) and cholesteryl hemisuccinate (CHEMS; Sigma-Aldrich) were dissolved in chloroform at concentrations of 2.1 and 0.45 mM, respectively, and the mixture was dried using a rotary evaporator under reduced pressure to obtain a lipid mixture. Meanwhile, an aqueous solution of siRNA having the following sequences (1.0 µM) and an 18% sucrose solution containing 290 nM poly-L-lysine hydrobromide (PLL; Sigma-Aldrich) were mixed in a volume ratio of 1:1, and the mixture was incubated at room temperature for 20 min to obtain an siRNA/PLL complex solution.
sense 5'- ACAUCACUUACGCUGAGUACUUCGA -AG - 3' (SEQ ID NO: 3)
antisense 3'-UA- UGUAGUGAAUGCGACUCAUGAAGCU - 5' (SEQ ID NO: 4)

This complex solution and D-MEM Medium (Sigma) were added to the above-mentioned lipid mixture, and the mixture was incubated at room temperature for 20 min and subjected to ultrasonic irradiation for 1 min with heating at approx. 65°C using a sonicator to obtain polyarginine-non-modified liposome dispersions having the concentrations shown in Table 3 (LP5, 6, 7, 8, 9, and 10). Then, LP5, 6, 7, 8, 9, and 10, respectively, were added to aqueous solutions of 3.8, 7.0, 28, 56, 112, and 224 µM R8-G-DOPE in a volume ratio of 13:1, and the mixtures were incubated at 37°C for 30 min to obtain polyarginine-modified liposome dispersions having the concentrations shown in Table 4 (Prescription Examples 5, 6, 7, 8, 9, and 10).

Meanwhile, controls for evaluations were prepared using water instead of the above-mentioned aqueous siRNA solution.

**[Table 3]**

| Polyarginine-non-modified liposome dispersion | LP5 | LP6 | LP7 | LP8 | LP9 | LP10 |
|---|---|---|---|---|---|---|
| DOPE concentration (µM) | 4.8 | 8.9 | 36 | 71 | 142 | 284 |
| CHEMS concentration (µM) | 1.1 | 2.0 | 7.8 | 16 | 31 | 62 |
| siRNA concentration (nM) | 80 | 80 | 80 | 80 | 80 | 80 |
| PLL concentration (nM) | 23 | 23 | 23 | 23 | 23 | 23 |

**[Table 4]**

| Polyarginine-modified liposome dispersion | Prescription Example 5 | Prescription Example 6 | Prescription Example 7 | Prescription Example 8 | Prescription Example 9 | Prescription Example 10 |
|---|---|---|---|---|---|---|
| DOPE concentration (µM) | 4.4 | 8.2 | 33 | 66 | 132 | 264 |
| CHEMS concentration (µM) | 1.0 | 1.8 | 7.2 | 14 | 29 | 58 |
| R8-G-DOPE concentration (µM) | 0.27 | 0.50 | 2.0 | 4.0 | 8 | 16 |
| siRNA concentration (nM) | 74 | 74 | 74 | 74 | 74 | 74 |
| PLL concentration (nM) | 22 | 22 | 22 | 22 | 22 | 22 |

### Preparation Example 3

Dioleoyl phosphatidyl ethanolamine (DOPE; NOF Corporation) and cholesteryl hemisuccinate (CHEMS; Sigma-Aldrich) were dissolved in chloroform at concentrations of 2.1 and 0.45 mM, respectively, and the mixture was dried using a rotary evaporator under reduced pressure to obtain a lipid mixture. Meanwhile, an aqueous solution of siRNA having the following sequences (1.0 µM) and an 18% sucrose solution containing 3.0 µM protamine sulfate (Sigma-Aldrich) were mixed in a volume ratio of 1:1, and the mixture was incubated at room temperature for 20 min to obtain an siRNA/protamine complex solution.
sense 5'- ACAUCACUUACGCUGAGUACUUCGA -AG - 3' (SEQ ID NO: 5)
antisense 3'-UA- UGUAGUGAAUGCGACUCAUGAAGCU - 5' (SEQ ID NO: 6)

This complex solution and D-MEM Medium (Sigma) were added to the above-mentioned lipid mixture, and the mixture was incubated at room temperature for 20 min and then subjected to ultrasonic irradiation using a sonicator for 1 min with heating at approx. 65°C to obtain polyarginine-non-modified liposome dispersions having the concentrations shown in Table 5 (LP11, 12, and 13). Then, aqueous R8-G-DOPE solutions at 3.8, 7.0, and 28 µM were added to LP11, 12, and 13, respectively, in a volume ratio of 13:1, and the mixtures were incubated at 37°C for 30 min to obtain polyarginine-modified liposome dispersions having the concentrations shown in Table 6 (Prescription Examples 11, 12, and 13).

Meanwhile, controls for evaluations were prepared using water instead of the above-mentioned aqueous siRNA solution.

**[Table 5]**

| Polyarginine-non-modified liposome dispersion | LP11 | LP12 | LP13 |
|---|---|---|---|
| DOPE concentration (µM) | 4.8 | 8.9 | 36 |
| CHEMS concentration (µM) | 1.1 | 2.0 | 7.8 |
| siRNA concentration (nM) | 80 | 80 | 80 |
| Protamine sulfate concentration (nM) | 236 | 236 | 236 |

**[Table 6]**

| Polyarginine-modified liposome dispersion | Prescription Example 11 | Prescription Example 12 | Prescription Example 13 |
|---|---|---|---|
| DOPE concentration (µM) | 4.4 | 8.2 | 33 |
| CHEMS concentration (µM) | 1.0 | 1.8 | 7.2 |
| R⁸-G-DOPE (µM) concentration | 0.27 | 0.50 | 2.0 |
| siRNA concentration (nM) | 74 | 74 | 74 |
| Protamine sulfate concentration (nM) | 219 | 219 | 219 |

### Preparation Example 4

Dioleoyl phosphatidyl ethanolamine (DOPE; NOF Corporation), dimyristoyl phosphatidylglycerol (DMPG; NOF Corporation), and cholesterol were dissolved in chloroform at concentrations of 1.7, 0.4, and 0.4 mM, respectively, and the mixture was dried using a rotary evaporator under reduced pressure to obtain a lipid mixture. Meanwhile, an aqueous solution of siRNA of the following sequences (1.0 µM) and an 18% sucrose solution containing 290 nM poly-L-lysine hydrobromide (PLL; Sigma-Aldrich) were mixed in a volume ratio of 1:1, and the mixture was incubated at room temperature for 20 min to obtain an siRNA/PLL complex solution.

sense 5'- ACAUCACUUACGCUGAGUACUUCGA -AG -
3' (SEQ ID NO: 7)

antisense 3'-UA- UGUAGUGAAUGCGACUCAUGAAGCU -
5' (SEQ ID NO: 8)

This complex solution and D-MEM Medium (Sigma) were added to the above-mentioned lipid mixture, and the mixture was incubated at room temperature for 20 min and then subjected to ultrasonic irradiation using a sonicator for 1 min with heating at approx. 65°C to obtain polyarginine-non-modified liposome dispersions having the concentrations shown in Table 7 (LP14, 15, 16, and 17). Then, aqueous solutions of 3.8, 7.0, 28, and 56 µM R8-G-DOPE were added to LP14, 15, 16, and 17, respectively, in a volume ratio of 13:1, and the mixtures were incubated at 37°C for 30 min to obtain polyarginine-modified liposome dispersions having the concentrations shown in Table 8 (Prescription Examples 14, 15, 16, and 17).

Meanwhile, controls for evaluations were prepared using water instead of the above-mentioned aqueous siRNA solution.

**[Table 7]**

| Polyarginine-non-modified liposome dispersion | LP14 | LP15 | LP16 | LP17 |
|---|---|---|---|---|
| DOPE concentration (µM) | 4.0 | 7.5 | 30 | 60 |
| DMPG concentration (µM) | 0.89 | 1.6 | 6.6 | 13 |
| Cholesterol concentration (µM) | 0.89 | 1.6 | 6.6 | 13 |
| siRNA concentration (nM) | 80 | 80 | 80 | 80 |
| PLL concentration (nM) | 23 | 23 | 23 | 23 |

**[Table 8]**

| Polyarginine-modified liposome dispersion | Prescription Example 14 | Prescription Example 15 | Prescription Example 16 | Prescription Example 17 |
|---|---|---|---|---|
| DOPE concentration (µM) | 3.7 | 7.0 | 28 | 56 |
| DMPG concentration (µM) | 0.82 | 1.5 | 6.1 | 12 |
| Cholesterol concentration (µM) | 0.82 | 1.5 | 6.1 | 12 |
| R8-G-DOPE concentration (µM) | 0.27 | 0.5 | 2.0 | 4.0 |
| siRNA concentration (nM) | 74 | 74 | 74 | 74 |
| PLL concentration (nM) | 22 | 22 | 22 | 22 |

### Test Example 1: Testing of siRNA transfection into CHO cells using Prescription Examples 1 to 4

20% FBS-containing F-12HAM Medium (Sigma) was added to Prescription Examples 1 to 4 and a control prescribed for evaluation in a volume ratio of 7:6 to obtain polyarginine-modified liposome/FBS-containing media. The medium of CHO (pMAM-luc) cells (JCRB0136.1, purchased from Health Science Research Resources Bank) was replaced with the polyarginine-modified liposome/FBS-containing media, and transfection was started. The cells were cultured at 37°C under 5.0% CO₂ for approx. 41 h, and the medium was replaced with F-12HAM Medium containing 1.0 µM dexamethasone and 10% FBS. The cells were cultured at 37°C under 5.0% CO₂ for approx. 6 to 8 h. Cells were observed under a microscope, and cytotoxicity was rated with scores (-, cells occupy approx. 85 to 100% of the visual field, and no trace of injury by toxicity is observed; ±, cells occupy approx. 85 to 100% of the visual field, but a trace of injury by toxicity is observed in some cells; +, cells occupy approx. 70 to 80% of the visual field; ++, cells occupy approx. 50 to 70% of the visual field; +++, cells occupy only less than approx. 50% of the visual field). The medium was removed, and the cells were washed with PBS. The cells were lysed with PLB, and then luciferase activity was measured. Furthermore, the knockdown rate (%) was calculated based on the equation (1). The results are shown in Table 9. Equation (1): 100 x (luciferase activity level after addition of siRNA/luciferase activity level without adding siRNA)

Separately, the knockdown rate was evaluated using Lipofectamine 2000 (trade name: Invitrogen Corporation) as a positive control.

The siRNA solution (1.0 pmol/µL) was 2.5-fold diluted with F-12HAM Medium to obtain a diluted siRNA solution. Separately, Lipofectamine 2000 was 50-fold diluted with F-12HAM Medium. Furthermore, the diluted siRNA solution was added to this solution in a volume ratio of 1:1 to obtain an siRNA/Lipofectamine 2000-containing medium. 20 µL of this medium was added to the separately cultured CHO (pMAM-luc) cell medium (100 µL), and transfection was started. The cells were cultured at 37°C under 5.0% CO₂ for approx. 41 h, and then the medium was replaced with F-12HAM Medium containing 1.0 µM dexamethasone and 10% FBS. The cells were cultured at 37°C under 5.0% CO₂ for approx. 6 to 8 h. Then the cells were observed under a microscope, and cytotoxicity was rated with scores (-, cells occupy approx. 85 to 100% of the visual field, and no trace of injury by toxicity is observed; ±, cells occupy approx. 85 to 100% of the visual field, but a trace of injury by toxicity is observed in some cells; +, cells occupy approx. 70 to 80% of the visual field; ++, cells occupy approx. 50 to 70% of the visual field; +++, cells occupy only less than approx. 50% of the visual field). The medium was removed, and the cells were washed with PBS. The cells were lysed with PLB, and then luciferase activity was measured. A similar experiment was performed as a control using water instead of the above-mentioned siRNA solution, and the knockdown rate (%) was calculated based on equation (1). The results are shown in Table 9.

### Test Example 2: Testing of siRNA transfection into HeLa cells by Prescription Examples 5 to 17

20% FBS-containing D-MEM Medium (Sigma) was added to Prescription Examples 5 to 17 and a control prescribed for evaluation in a volume ratio of 7:6 to obtain polyarginine-modified liposome/FBS-containing media. These polyarginine-modified liposome/FBS-containing media were replaced with the medium of NFAT Reporter HeLa Stable Cell Line (Panomics), and transfection was started. The cells were cultured at 37°C under 5.0% CO₂ for approx. 18 h, and then the medium was replaced with D-MEM Medium containing 10 ng/mL PMA, 0.50 µM Calcium Ionophore A23187, and 10% FBS. The cells were cultured at 37°C under 5.0% CO₂ for approx. 6 h. Then the cells were observed under a microscope, and cytotoxicity was rated with scores (-, cells occupy approx. 85 to 100% of the visual field, and no trace of injury by toxicity is observed; ±, cells occupy approx. 85 to 100% of the visual field, but a trace of injury by toxicity is observed in some cells; +, cells occupy approx. 70 to 80% of the visual field; ++, cells occupy approx. 50 to 70% of the visual field; +++, cells occupy only less than approx. 50% of the visual field). The medium was removed, and then the cells were washed with PBS. The cells were lysed with PLB, and then the luciferase activity was measured.

Furthermore, the knockdown rate (%) was calculated based on equation (1). The results are shown in Tables 10 and 11.

Separately, the knockdown rate was evaluated using Lipofectamine 2000 (trade name: Invitrogen Corporation) as a positive control.

The siRNA solution (1 pmol/µL) was 2.5-fold diluted with D-MEM Medium to obtain a diluted siRNA solution. Separately, Lipofectamine 2000 was 100-fold diluted with a D-MEM medium. Furthermore, the diluted siRNA solution was added to this solution in a volume ratio of 1:1 to obtain an siRNA/Lipofectamine 2000-containing medium. 20 µL of this medium was added to separately-cultured NFAT Reporter HeLa Stable Cell Line Medium (100 µL), and transfection was started. The cells were cultured at 37°C under 5.0% CO₂ for approx. 18 h, and then the medium was replaced with D-MEM Medium containing 10 ng/mL PMA, 0.50 µM Calcium Ionophore A23187, and 10% FBS. The cells were cultured at 37°C under 5.0% CO₂ for approx. 6 h. Then, the cells were observed under a microscope, and cytotoxicity was rated with scores (-, cells occupy approx. 85 to 100% of the visual field, and no trace of injury by toxicity is observed; ±, cells occupy approx. 85 to 100% of the visual field, but a trace of injury by toxicity is observed in some cells; +, cells occupy approx. 70 to 80% of the visual field; ++, cells occupy approx. 50 to 70% of the visual field; +++, cells occupy only less than approx. 50% of the visual field). The medium was removed, and then cells were washed with PBS. The cells were lysed with PLB, and luciferase activity was measured. A similar experiment of a control was performed using water instead of the above-mentioned siRNA solution, and the knockdown rate (%) was calculated based on equation (1). The results are shown in Tables 10 and 11.

As shown in Tables 9 to 11, the compositions of the present invention exhibited excellent nucleic acid introduction efficiency (knockdown rate) and weak cytotoxicity.

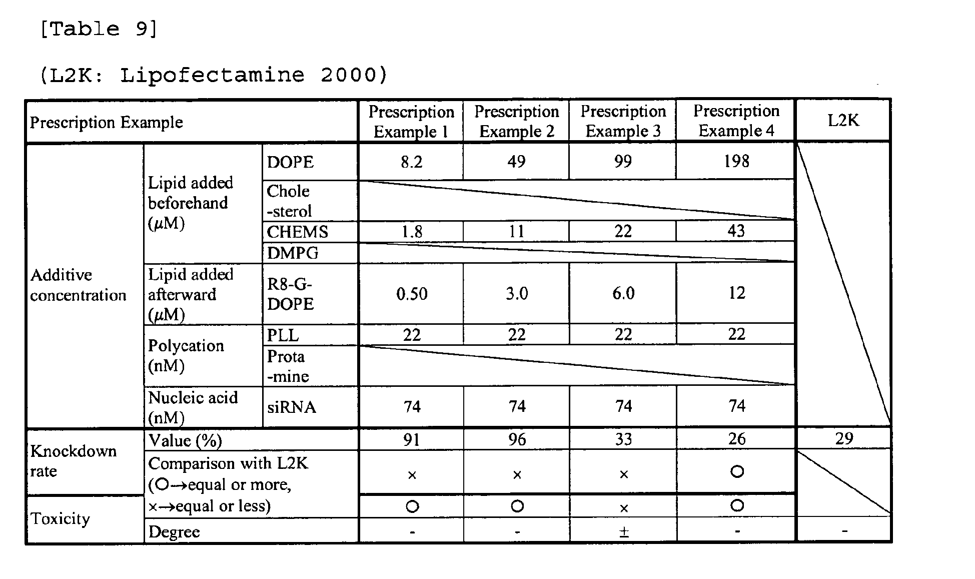

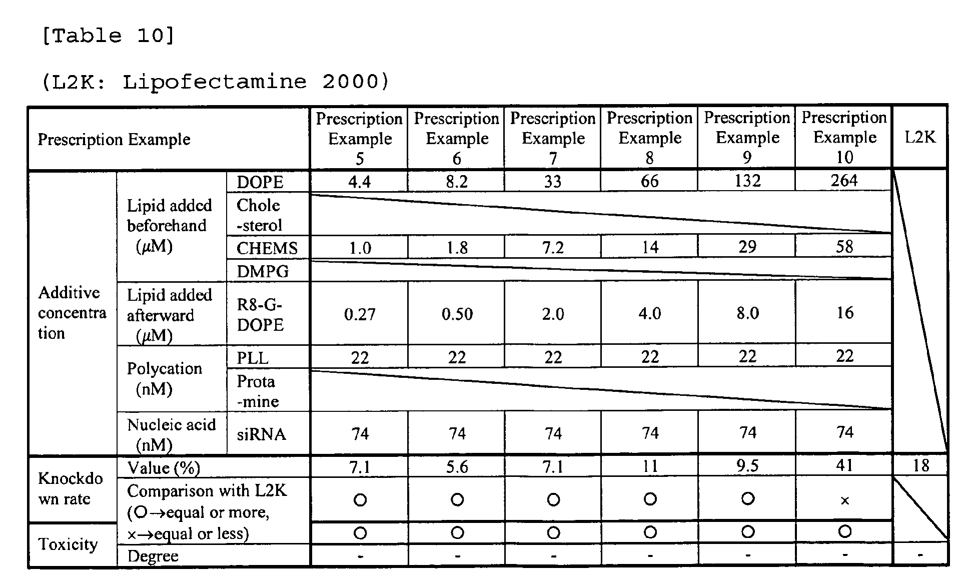

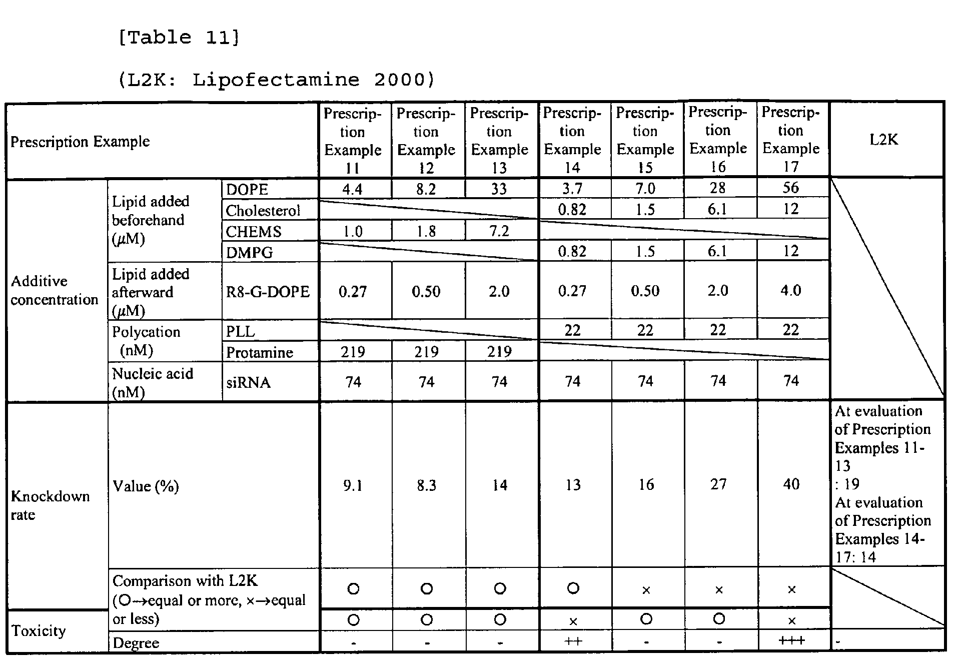

### Industrial Applicability

A nucleic acid can be efficiently introduced with weak cytotoxicity using the composition of the present invention, and the composition of the present invention is useful as a reagent or a medicament for introducing a nucleic acid.

### [Sequence listing free text]

SEQ ID NO: 1: Sense RNA constituting siRNA against luciferase
SEQ ID NO: 2: Antisense RNA constituting siRNA against luciferase
SEQ ID NO: 3: Sense RNA constituting siRNA against luciferase
SEQ ID NO: 4: Antisense RNA constituting siRNA against luciferase
SEQ ID NO: 5: Sense RNA constituting siRNA against luciferase
SEQ ID NO: 6: Antisense RNA constituting siRNA against luciferase
SEQ ID NO: 7: Sense RNA constituting siRNA against luciferase
SEQ ID NO: 8: Antisense RNA constituting siRNA against luciferase

## Claims

1. A compound represented by formula (I):
(R¹)n -R²-R³ (I)
wherein: (R¹)n represents a polyamino acid residue consisting of a total of n amino acid residues, which are identical to or different from one another, the n residues being selected from the group consisting of an arginine residue, a lysine residue, and a histidine residue, and n being an integer of from 4 to 16; R² represents a single bond or an amino acid residue; and R³ represents a phospholipid residue with 1 to 4 identical or different unsaturated fatty acid residues having from 12 to 20 carbon atoms or a salt thereof.

2. The compound or a salt thereof according to claim 1, wherein R¹ are all arginine residues.

3. The compound or a salt thereof according to claim 1 or 2, wherein n is from 7 to 11.

4. The compound or a salt thereof according to claim 1 or 2, wherein n is from 8 to 10.

5. The compound or a salt thereof according to any one of claims 1 to 4, wherein R² is a glycine residue.

6. The compound or a salt thereof according to any one of claims 1 to 5, wherein R³ is a dioleoyl phosphatidyl ethanolamine residue.

7. A composition comprising the compound or a salt thereof according to any one of claims 1 to 6 and a lipid.

8. The composition according to claim 7, wherein the lipid is a phospholipid and/or a sterol.

9. The composition according to claim 8, wherein the phospholipid is one or more selected from phosphatidyl ethanolamines, phosphatidylcholines, phosphatidylserines, phosphatidylinositols, phosphatidylglycerols, cardiolipins, sphingomyelins, plasmalogens, and phosphatidic acids.

10. The composition according to claim 8, wherein the phospholipid is a phosphatidyl ethanolamine.

11. The composition according to claim 8, wherein the phospholipid is a dioleoyl phosphatidyl ethanolamine.

12. The composition according to any one of claims 8 to 11, wherein the sterol is a cholesterol.

13. The composition according to any one of claims 7 to 12, further comprising a cationic substance.

14. The composition according to claim 13, wherein the cationic substance is one or more selected from poly L-lysine, protamine or a salt thereof, pronectin, and spermine.

15. The composition according to any one of claims 7 to 14, for introducing a nucleic acid into a cell.

16. The composition according to any one of claims 7 to 15, further comprising a nucleic acid.

17. The composition according to any one of claims 7 to 16, which forms a lipid membrane structure.

18. The composition according to claim 17, wherein the lipid membrane structure is a liposome.

19. A method for introducing a nucleic acid into a cell, **characterized in that** the composition according to any one of claims 16 to 18 is applied to a cell *in vitro* or *in vivo*.


### [Sequence Listing]

SEQUENCE LISTING

<110>  Daiichi Sankyo Company, Limited

<120>  Composition for Nucleic Acid Transfer

<130>  EPP102079

<140>  EP07831200.6
<141>  2007-11-05

<150>  JP2006-304591

<151>  2006-11-09
<160>  8

<170>  PatentIn version 3.1

<210>  1
<211>  27
<212>  RNA
<213>  Artificial sequence

<220>
<223>  siRNA(sense sequence)

<400>  1
acaucacgua cgcggaauac uucgaag                                          27


<210>  2
<211>  27
<212>  RNA
<213>  Artificial sequence

<220>
<223>  siRNA(antisense sequence)

<400>  2
uauguagugc augcgccuua ugaagcu                                          27


<210>  3
<211>  27
<212>  RNA
<213>  Artificial sequence

<220>
<223>  siRNA(sense sequence)

<400>  3
acaucacuua cgcugaguac uucgaag                                          27


<210>  4
<211>  27
<212>  RNA
<213>  Artificial sequence

<220>
<223>  siRNA(antisense sequence)

<400>  4
uauguaguga augcgacuca ugaagcu                                          27


<210>  5
<211>  27
<212>  RNA
<213>  Artificial sequence

<220>
<223>  siRNA(sense sequence)

<400>  5
acaucacuua cgcugaguac uucgaag                                          27


<210>  6
<211>  27
<212>  RNA
<213>  Artificial sequence

<220>
<223>  siRNA(antisense sequence)

<400>  6
uauguaguga augcgacuca ugaagcu                                          27


<210>  7
<211>  27
<212>  RNA
<213>  Artificial sequence

<220>
<223>  siRNA(sense sequence)

<400>  7
acaucacuua cgcugaguac uucgaag                                          27


<210>  8
<211>  27
<212>  RNA
<213>  Artificial sequence

<220>
<223>  siRNA(antisense sequence)

<400>  8
uauguaguga augcgacuca ugaagcu                                          27